# EUROPEAN PATENT SPECIFICATION

(11) **EP 2 653 147 B1**
(45) Date of publication and mention of the grant of the patent: **31.08.2016**
(21) Application number: 11848121.7
(22) Date of filing: 09.09.2011
(51) Int. Cl.: A61N 5/06, A61H 39/06

(54) **LASER AND INFRARED PHOTON ACUPUNCTURE INSTRUMENT OUTPUT HEAD AND ACUPUNCTURE INSTRUMENT**
AUSGABEKOPF FÜR LASER- UND INFRAROT-PHOTONEN-AKUPUNKTURINSTRUMENT SOWIE AKUPUNKTURINSTRUMENT
TÊTE DE SORTIE D'INSTRUMENT D'ACUPUNCTURE ET DE MOXIBUSTION À LASER ET PHOTONS INFRAROUGES, ET INSTRUMENT D'ACUPUNCTURE ET DE MOXIBUSTION

(30) Priority: 14.12.2010 CN 201020658513 U
(43) Date of publication of application: 23.10.2013
(73) Proprietor: Advance Technology Limited, Shenzhen, Guangdong 518000 (CN)
(72) Inventor: Lee Yiu Lam, Shenzhen, Guangdong 518000 (CN)
(74) Representative: ProI European Patent Attorneys
(86) International application number: PCT/CN2011/079538
(87) International publication number: WO 2012/079395

(56) References cited:
- EP-A1- 1 262 210
- EP-A2- 1 854 505
- WO-A1-02/062419
- WO-A1-2007/119946
- CN-A- 1 294 025
- CN-A- 101 134 128
- CN-U- 201 880 012
- CN-Y- 2 566 876
- DE-A1- 3 729 288
- DE-A1- 19 929 714
- JP-A- 2000 153 003
- US-A- 4 553 546

## Description

### Technical Field

The invention relates to a medical healthcare product, in particular to an output head of an acupuncture instrument integrating laser and infrared photon.

### Background of the Invention

Various lasers, such as helium neon lasers, carbon dioxide lasers, semiconductor lasers, ionic lasers, Nd-YAG lasers (Nd.YAG for short, from Neodymium-doped Yttrium Aluminium Garnet; Nd: Y3Al5O12) have been widely applied to clinic successively. Further devices for medical and/or cosmetic use have been proposed e.g. in documents EP 1 262 210 A1, JP 2000-153003, EP 1 854 505 A2 and DE 37 29 288 A1.

The lasers described above have the following problems.
1) Helium neon lasers and carbon dioxide lasers are rarely suitable for household use due to large size and high price, and also difficult in miniaturization.
2) Semiconductor lasers are widely applied to acupuncture and easy to be miniaturized, but with the absence of optical moxibustion function and mono therapeutic effect.
3) The present moxibustion instruments of optical moxibustion, due to their narrow spectral bands or simulation of moxibustion with single wavelength infrared laser, are quite different from the broad spectrum infrared bands of moxibustion.
4) The moxibustion treatment mainly shows comprehensively stimulatory effect at the broad spectrum of the near-infrared band and the far-infrared band. The present moxibustion instruments of optical moxibustion have poor therapeutic effect as they have no broad spectrum infrared spectral bands.

It is expected to provide a laser and infrared photon acupuncture instrument, in order to solve the problem in the prior art of poor combination of miniaturization and therapeutic effect of lasers. The invention provides improvements on heat sinking capacity of the laser and infrared photon acupuncture instrument output head, and on connection of the output head to the protecting band.

### Summary of the Invention

In order to solve the problem in the prior art, the invention provides a laser and infrared photon acupuncture instrument output head, the output head comprising a front housing, a middle housing and a rear housing sequentially connected, the output head also comprising therein an infrared PCB assembly disposed at the front end thereof and a laser PCB assembly disposed at the rear end thereof, and a heat sink being provided between the infrared PCB assembly and the laser PCB assembly. The laser and infrared photon acupuncture instrument output head further comprises a thermistor provided at the front end of the output head.

As a further improvement of the invention, the heat sink is a metal heat sink.

As a further improvement of the invention, the front end of the front housing is provided with a protective mesh.

As a further improvement of the invention, the periphery of the front housing is provided with flexible pieces.

As a further improvement of the invention, there are 3 to 5 flexible pieces, which are evenly provided on the periphery of the front housing.

The invention also provides a laser and infrared photon acupuncture instrument, the acupuncture instrument is provided with any of the described laser and infrared photon acupuncture instrument output head.

The laser and infrared photon acupuncture instrument output head of the invention is added with a metal heat sink having rapid cooling effect, thereby prolonging the product service life.

The protective mesh is used for preventing a person (especially children) from inserting a hand thereof and being burned by a bulb.

In the aspect of connection to a strap: several (preferably four) small flexible pieces are provided on the front housing, and corresponding grooves are provided on an attachment connected to the strap, allowing a convenient plugging and unplugging of the output head.

### Brief Description of the Drawings

Fig. 1 is a structure diagram of a laser and infrared photon acupuncture instrument of the invention;
Fig. 2 is a structure diagram of a laser and infrared photon acupuncture instrument output head of the invention; and,
Fig. 3 is an enlarged structure diagram of part A of Fig. 2.

### Detailed Description of the Invention

The invention will be further described as below by specific embodiments with reference to drawings.

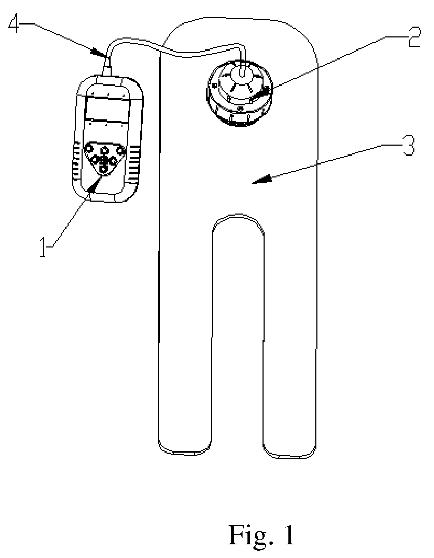

As shown in Fig. 1, a laser and infrared photon acupuncture instrument is provided, comprising a controller 1, an output head 2, a protective band 3 and a cable 4. The controller 1 is connected to the output head 2 via the cable 4. The output head 2 is connected with the protective band 3 by pressing the attachment connected to the strap by the flexible pieces. The protective band 3 is tied to the position to be treated. The controller 1 is handheld to control the output head 2 on the protective band 3 to work.

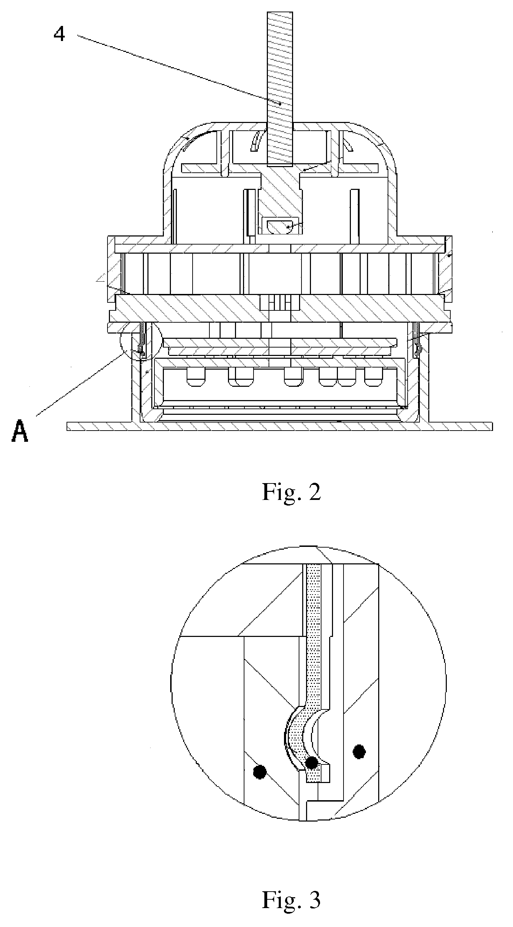

As shown in Fig. 2 and Fig. 3, the output head integrates laser with infrared photon, with infrared photon at the front end and laser at the rear end. Laser is emitted to the front from a through hole in the middle, to operate treatment on the location of lesion together with the infrared photon. In order to prevent heat radiated by the infrared photon from affecting the effect of laser, an insulating plate and a heat sink (preferably metal heat sink) are thus added between the laser PCB and the infrared PCB, with many heat dissipation holes added therebetween. In order to protect a person from being burned, a thermistor is additionally provided at the front end of the product to control over-high temperature. The front end of the front housing of the output head is provided with a protective mesh which is also used for preventing a person from inserting a hand thereof into the infrared tube and being burned. Four small flexible pieces are additionally provided on the front housing, and corresponding grooves are provided on an attachment connected to the strap, allowing a convenient plugging and unplugging of the output head.

The above descriptions are further detailed descriptions of the invention by preferred embodiments. It will not be understood that the specific implementation of the invention is limited to the descriptions.

## Claims

1. A laser and infrared photon acupuncture instrument output head (2), wherein the output head (2) includes a front housing, a middle housing and a rear housing sequentially connected, and the output head (2) also includes therein an infrared printed circuit board (PCB) assembly disposed at the front end thereof and a laser PCB assembly disposed at the rear end thereof, a heat sink being provided between the infrared PCB assembly and the laser PCB assembly; further including a thermistor at the front end of the output head (2).

2. The laser and infrared photon acupuncture instrument output head (2) according to claim 1, wherein the heat sink is a metal heat sink.

3. The laser and infrared photon acupuncture instrument output head (2) according to claim 1 or 2, wherein the front end of the front housing is provided with a protective mesh.

4. The laser and infrared photon acupuncture instrument output head (2) according to any one of claims 1 to 3, wherein the periphery of the front housing is provided with flexible pieces.

5. The laser and infrared photon acupuncture instrument output head (2) according to claim 4, having 3 to 5 flexible pieces, which are evenly provided on the periphery of the front housing.

6. A laser and infrared photon acupuncture instrument, wherein the acupuncture instrument is provided with the laser and infrared photon acupuncture instrument output head (2) according to any one of claims 1 to 5.

## Patentansprüche

1. Laser- und Infrarot-Photonen-Akupunkturinstrument-Ausgabekopf (2), wobei der Ausgabekopf (2) ein vorderes Gehäuse, ein mittleres Gehäuse und ein hinteres Gehäuse der Reihe umfasst, welche in Reihe miteinander verbunden sind, und der Ausgabekopf (2) umfasst darin auch ein Infrarot-Leiterplatten (PCB)- Anordnung, welche am vorderen Ende davon angeordnet ist, und eine Laser-Leiterplatten (PCB)-Anordnung, welche am hinteren Ende davon angeordnet ist, wobei ein Kühlkörper zwischen der Infrarot-PCB-Anordnung und der Laser-PCB-Anordnung vorgesehen ist, ferner umfassend einen Thermistor am vorderen Ende des Ausgabekopfes (2).

2. Laser- und Infrarot-Photonen-Akupunkturinstrument-Ausgabekopf (2) nach Anspruch 1, wobei der Kühlkörper ein Metallkühlkörper ist.

3. Laser- und Infrarot-Photonen-Akupunkturinstrument-Ausgabekopf (2) nach Anspruch 1 oder 2, wobei das vordere Ende des vorderen Gehäuses mit einem Schutzgitter versehen ist.

4. Laser- und Infrarot-Photonen-Akupunkturinstrument-Ausgabekopf (2) nach einem der Ansprüche 1 bis 3, wobei der Umfang des vorderen Gehäuses mit flexiblen Stücken versehen ist.

5. Laser- und Infrarot-Photonen-Akupunkturinstrument-Ausgabekopf (2) nach Anspruch 4, mit 3 bis 5 flexiblen Stücken, die am Umfang des vorderen Gehäuses gleichmäßig verteilt vorgesehen sind.

6. Laser- und Infrarot-Photonen-Akupunkturinstrument, wobei das Akupunkturninstrument mit einem Laser- und Infrarot-Photonen-Akupunkturinstrument-Ausgabekopf (2) gemäß einem der Ansprüche 1 bis 5 versehen ist.

## Revendications

1. Une tête de sortie (2) laser et photon infrarouge instrument d'acupuncture selon, dans lequel la tête de sortie (2) comprend un boîtier avant, un boîtier médiane et une séquence de boîtier arrière est connecté, et la tête de sortie comprend également à l'intérieur un assemblage de circuits imprimés à infrarouge disposé à l'extrémité avant de celui-ci et un circuit imprimé par laser ensemble disposé à l'extrémité arrière de celui-ci, un dissipateur de chaleur étant prévu entre l'ensemble de PCB infrarouge et l'assemblage de circuits imprimés au laser, dans une thermistance est en outre prévu à l'extrémité avant du la tête de sortie (2).

2. La tête de sortie (2) laser et photon infrarouge instrument d'acupuncture selon la revendication 1, dans lequel le dissipateur de chaleur est un dissipateur thermique en métal.

3. La tête de sortie (2) laser et photon infrarouge instrument d'acupuncture selon la revendication 1 ou 2, dans lequel l'extrémité avant du boîtier avant est munie d'un filet de protection.

4. La tête de sortie (2) laser et photon infrarouge instrument d'acupuncture selon l'une quelconque des revendications 1 à 3, dans lequel la périphérie du boîtier avant est pourvue d'éléments flexibles.

5. La tête de sortie (2) laser et photon infrarouge instrument d'acupuncture selon la revendication 4, dans lequel il y a 3 à 5 pièces souples, qui sont régulièrement disposées sur la périphérie du boîtier avant.

6. Un laser et infrarouge instrument photon d'acupuncture, dans lequel l'instrument d'acupuncture est fourni avec la tête de sortie (2) laser et photon infrarouge instrument d'acupuncture selon l'une quelconque des revendications 1 à 5.
